# EUROPEAN PATENT APPLICATION

(11) **EP 4 467 090 A1**
(43) Date of publication of application: **27.11.2024**
(21) Application number: 24173759.2
(22) Date of filing: 02.05.2024
(51) Int. Cl.: A61B 18/12, A61N 1/00

(54) **AMPLITUDE MODULATION TO ASSESS CATHETER ENVIRONMENT**

(30) Priority: 23.05.2023 US 202363503886 P
(71) Applicant: Medtronic Ireland Manufacturing Unlimited Company, Dublin 2, D02 T380 (IE)
(72) Inventor: ANDERSON, Charles J., Blaine, 55434 (US)
(74) Representative: Zimmermann & Partner Patentanwälte mbB

(57) **Abstract**

A system may modulate a carrier signal to generate a modulated signal that carries a message for transmission by a first electrode of a catheter. The system may receive an indication of the modulated signal received by a second electrode of the catheter. The system may demodulate the modulated signal received by the second electrode to extract a demodulated message. The system may determine whether the first electrode contacts a target tissue site of a patient based at least in part on the demodulated message.

## Description

This application claims the benefit of U.S. Provisional Patent Application Serial No. 63/503,886, filed May 23, 2023, the entire content of which is incorporated herein by reference.

### TECHNICAL FIELD

The present disclosure relates to medical catheters.

### BACKGROUND

A medical catheter including electrodes may be used for therapeutic applications, such as ablation applications, electrical simulation therapy, and the like.

### SUMMARY

In general, aspects of this disclosure describes techniques for assessing the environment of a medical catheter by performing modulation of signals delivered by electrodes of the medical catheter. For example, a medical system may perform amplitude modulation of a carrier signal, such as by mixing or concatenating the carrier signal with a message signal to generate a modulated signal that carries the message signal. A first electrode of the medical catheter transmits the modulated signal carrying the message signal. The first electrode may deliver the modulated signal and a second electrode of the medical catheter may receive the modulated signal.

Because the modulated signal delivered by the first electrode may pass through a medium, such as walls of an organ or vessel, fat, and/or blood before being received by the second electrode, the modulated signal may suffer loss as the signal passes through such a medium. The modulated signal as received by the second electrode may therefore be different from the modulated signal transmitted by the first electrode. The medical system may demodulate the modulated signal received by the second electrode to extract, from the received modulated signal, a demodulated message, and may compare the demodulated message extracted from the modulated signal received by the second electrode with the message carried by the modulated signal transmitted by the first electrode to determine whether the medical catheter is in contact with an organ tissue, arterial walls, venous walls, or other structures.

In some aspects, the techniques described herein relate to a method including: modulating, by processing circuitry, a carrier signal to generate a modulated signal that carries a message for transmission by a first electrode of a catheter; receiving, by the processing circuitry, an indication of the modulated signal received by a second electrode of the catheter; demodulating, by the processing circuitry, the modulated signal received by the second electrode to extract a demodulated message; and determining, by the processing circuitry, whether the first electrode contacts a target tissue site of a patient based at least in part on the demodulated message.

In some aspects, the techniques described herein relate to a system including: memory; processing circuitry configured to: modulate a carrier signal to generate a modulated signal that carries a message for transmission by a first electrode of a catheter; receive an indication of the modulated signal received by a second electrode of the catheter; demodulate the modulated signal received by the second electrode to extract a demodulated message; and determine whether the first electrode contacts a target tissue site of a patient based at least in part on the demodulated message.

In some aspects, the techniques described herein relate to an apparatus including: means for modulating a carrier signal to generate a modulated signal that carries a message for transmission by a first electrode of a catheter; means for receiving an indication of the modulated signal received by a second electrode of the catheter; means for demodulating the modulated signal received by the second electrode to extract a demodulated message; and means for determining whether the first electrode contacts a target tissue site of a patient based at least in part on the demodulated message.

In some aspects, the techniques described herein relate to a non-transitory computer readable storage medium including instructions that, when executed by processing circuitry, cause the processing circuitry to: modulate a carrier signal to generate a modulated signal that carries a message for transmission by a first electrode of a catheter; receive an indication of the modulated signal received by a second electrode of the catheter; demodulate the modulated signal received by the second electrode to extract a demodulated message; and determine whether the first electrode contacts a target tissue site of a patient based at least in part on the demodulated message.

Further disclosed herein is a system that may modulate a carrier signal to generate a modulated signal that carries a message for transmission by a first electrode of a catheter, wherein the system may receive an indication of the modulated signal received by a second electrode of the catheter, wherein the system may demodulate the modulated signal received by the second electrode to extract a demodulated message, and wherein the system may determine whether the first electrode contacts a target tissue site of a patient based at least in part on the demodulated message.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

Reference is made to the attached drawings, wherein elements having the same reference numeral designations represent similar elements throughout and wherein:
FIG. 1 is a schematic and conceptual illustration of an example system for determining whether a catheter is in contact with a target tissue site.
FIG. 2 illustrates using pairs of electrodes of a catheter to determine whether the catheter is in contact with a target tissue site.
FIG. 3 is a flow diagram illustrating an example technique for determining appositional information associated with a catheter.

### DETAILED DESCRIPTION

Medical catheters include electrodes used to deliver therapy, such as ablation therapy. For example, the electrodes may deliver high-frequency electrical current to targeted tissue to destroy or damage the tissue in a controlled manner to treat a variety of medical conditions, such as abnormal heart rhythms, tumors, or chronic pain. The electrodes may be disposed on any suitable portion of an elongated member of a catheter, and the catheter is configured to electrically connect the electrodes to a medical device, which may deliver electrical signals and/or radio frequency signals to a tissue site within a patient via one or more of the electrodes.

In order to safely and effectively deliver therapy, such as ablation therapy, to a targeted area, such as a tissue area, the medical catheter is apposed to a target area. That is, one or more electrodes of the medical catheter may contact the target area, such as a target tissue site on an organ tissue, arterial walls, venous walls, or other structures in order to deliver ablation therapy to the target tissue site.

Techniques for determining whether an electrode of a medical catheter is in contact with a target tissue site may include using imaging of a patient or live x-rays to determine whether the electrode of the medical catheter is in contact with the target tissue. However, the use of imaging or live x-rays may require additional equipment. Techniques for determining whether an electrode of a medical catheter is in contact with a target tissue site may also include measuring the impedance and/or amplitude of electrical signals delivered by the electrode to determine changes to the impedance and/or amplitude of electrical signals due to organ tissue, arterial walls, venous walls, or other structures. However, such techniques may potentially be unreliable as changes to the impedance and/or amplitude of electrical signals due to organ tissue, arterial walls, venous walls, or other structures may vary from patient to patient and may not provide a stable or clear measure of whether the electrode is in contact with a target tissue site.

In accordance with aspects of this disclosure, a medical device or system may perform modulation (e.g., amplitude modulation) of electrical signals delivered by one or more electrodes of a medical catheter to assess the environment of the medical catheter, such as to determine whether the one or more electrodes of the medical catheter are in contact with an organ tissue, arterial walls, venous walls, or other structures in order to deliver ablation therapy to a targeted tissue site. The medical device or system may perform modulation of a carrier signal, such as by mixing or concatenating the carrier signal with a message signal to generate a modulated signal that carries the message signal that is transmitted by an electrode of the medical catheter. A first electrode of the medical catheter may deliver the modulated signal in the form of electrical energy, and a second electrode of the medical catheter may receive the modulated signal.

Because the modulated signal delivered by the first electrode may pass through a medium, such as walls of an organ or vessel, fat, and/or blood before being received by the second electrode, the modulated signal may suffer loss as the signal passes through such a medium, and the modulated signal as received by the second electrode may therefore be different from the modulated signal transmitted by the first electrode. As such, the medical device or system may be able to determine the medium through which the modulated signal has passed based on the difference between the modulated signal transmitted by the first electrode and the modulated signal received by the second electrode.

The medical device or system may demodulate the modulated signal received by the second electrode to extract, from the received modulated signal, a demodulated message, and may compare the demodulated message extracted from the modulated signal received by the second electrode with the message carried by the modulated signal transmitted by the first electrode to determine whether the medical catheter is in contact with an organ tissue, arterial walls, venous walls, or other structures. For example, the medical device or system may compare the demodulated message extracted from the modulated signal received by the second electrode with the message carried by the modulated signal transmitted by the first electrode to determine the recovery rate of the message carried by the demodulated message. In some examples, a relatively high recovery rate may indicate that the medical catheter is not in contact with an organ tissue, arterial walls, venous walls, or other structures, while a relatively low recovery rate may indicate that the medical catheter is in contact with an organ tissue, arterial walls, venous walls, or other structures. In this way, the techniques of this disclosure may enable the medical device or system to more reliably determine whether the medical catheter is in contact with a target tissue site and possibly without the use of additional equipment such as imaging equipment or x-ray equipment.

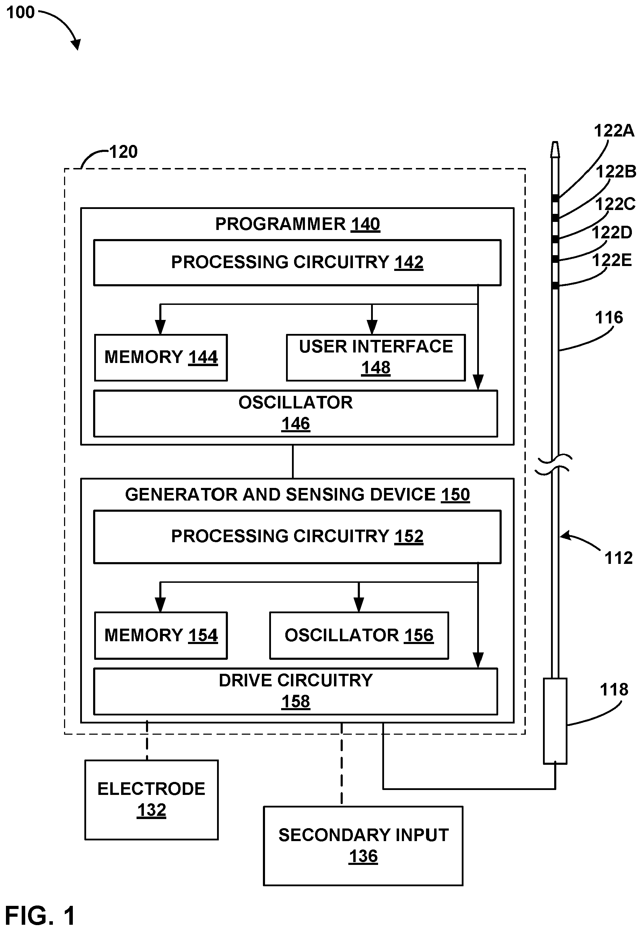

FIG. 1 illustrates an example system 100 for determining whether a catheter is in contact with a target tissue site. As shown in FIG. 1, system 100 includes catheter 112 coupled to generator and sensing device 150. Catheter 112 includes electrodes 122A-122E ("electrodes 122") used to deliver therapy, such as ablation therapy, to a target tissue site, such as an organ tissue, arterial walls, venous walls, or other structures of a patient. In some examples, catheter 112 may include a hub portion 118 coupled to a proximal portion of elongated member 116 of catheter 112. Electrodes 122 may be disposed in any arrangement on elongated member 116. In some examples, electrodes 122 are positioned at a distal portion of elongated member 116, as shown in FIG. 1. In other examples, electrodes 122 may be positioned elsewhere on elongated member 116. While FIG. 1 illustrates catheter 112 having five electrodes 122A-122E, catheter 112 may, in other examples, include any other number of electrodes, such as six, seven, or eight electrodes.

Catheter 112 (e.g., via elongated member 116) may include at least one electrical conductor for electrically coupling electrodes 122 to generator and sensing device 150. In some examples, each electrode of electrodes 122 may be coupled to a respective electrical conductor. In other examples, two or more electrodes of electrodes 122 may be electrically coupled to the same electrical conductor. Thus, catheter 112 is configured to receive one or more electrical signals from generator and sensing device 150 and to deliver the electrical signal to a tissue site via one or more of electrodes 122.

Generator and sensing device 150 is configured to generate and deliver an electrical signal (for example, via a least one electrical conductor within elongated member 116) to one or more of electrodes 122, so that electrodes 122 may deliver energy to a target tissue site within a patient. The energy may include electrical energy, radiofrequency (RF) energy, pulsed electrical energy, thermal energy, or the like. Electrodes 122 may deliver therapy to the target tissue site of the patient. Such therapy may include electrical stimulation therapy, such as cardiac therapy or neurostimulation therapy, ablation, or denervation, such as renal denervation. Generator and sensing device 150 may include any suitable medical device and/or computing device configured to generate and deliver an electrical signal that may provide therapeutic benefits to a patient, such as an ablation device, a neurostimulation device, a cardiac stimulation device, or another electrical stimulation device.

In some examples, generator and sensing device 150 includes an electrical source, for example, an RF energy generator. Generator and sensing device 150 is configured to generate a selected form and magnitude of energy for delivery to the target treatment site via electrodes 122. For example, generator and sensing device 150 may include a processing circuitry 152 configured to select one or more of electrodes 122 from which electrical signals are delivered, and may include drive circuitry 158 for electrically driving electrodes 122. Drive circuitry 158 may amplify or send electrical signals from processing circuitry 152 to electrodes 122.

Generator and sensing device 150 may include a memory 154. Memory 154 includes computer-readable instructions that, when executed by processing circuitry 152, causes generator and sensing device 150 to perform various functions. Processing circuitry 152 may include any one or more microprocessors, controllers, digital signal processors (DSPs), application specific integrated circuits (ASICs), field-programmable gate arrays (FPGAs), or equivalent discrete or integrated digital or analog logic circuitry, and the functions attributed to processing circuitry 152 herein may be embodied as software, firmware, hardware or any combination thereof.

Memory 154 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media. Memory 154 may store any suitable information, including patient identification information, and information for selecting one or more electrodes through which generator and sensing device 150 generates and delivers therapy to a patient. Memory 154 may also store operating instructions with which processing circuitry 152 controls the operation of generator and sensing device 150.

In some examples, electrodes 122 are configured to be electrically connected to a medical device in series or in parallel. When connected in series, e.g., via respective electrical conductors, therapy generator and sensing device 150 may deliver electrical signals via all the electrodes of electrodes 122 or via a selected subset of electrodes of electrodes 122, which can include one electrode or multiple electrodes. In some examples, processing circuitry 152 of therapy generator and sensing device 150 may be configured to select a subset of electrodes 122 for delivery of electrical energy, and may make the selection in response to user input or in response to a predetermined therapy program stored by memory. When connected in parallel, therapy generator and sensing device 150 may deliver an electrical signal via all the electrodes of electrodes 122 simultaneously.

In some examples, catheter 112 includes at least a first electrical conductor and a second electrical conductor. The first and the second electrical conductors may extend within elongated member 116 from a proximal portion of catheter 112 to electrodes 122. The first electrical conductor may be configured to deliver a first electrical signal from therapy generator and sensing device 150 to at least one of electrodes 122. The second electrical conductor may be configured to deliver a second electrical signal from therapy generator and sensing device 150 to at least one of electrodes 122.

In some examples, catheter 112 may include a plurality of electrical conductors extending within elongated member 116 from a proximal portion of catheter 112 to electrodes 122. The plurality of electrical conductors may include the first electrical conductor and the second electrical conductor. Each electrical conductor of the plurality of electrical conductors may be configured to deliver a respective electrical signal of a plurality of electrical signals from therapy generator and sensing device 150 to a respective electrode of electrodes 122. The plurality of electrical signals may include the first electrical signal and the second electrical signal.

Electrodes 122 may be carried by or disposed on elongated member 116. Elongated member 116 is configured to be navigated through vasculature of the patient, for example, to position electrodes 122 adjacent the target treatment site of the patient. For example, elongated member 116 may be used to access relatively distal vasculature locations in a patient or other relatively distal tissue sites (e.g., relative to a vasculature access point). Example vasculature locations may include, for example, locations in a coronary artery, peripheral vasculature (e.g., carotid, iliac, or femoral artery, or a vein), or cerebral vasculature. In some examples, elongated member 116 is structurally configured to be relatively flexible, pushable, and relatively kink- and buckle- resistant, so that it may resist buckling when a pushing force is applied to a relatively proximal portion of catheter 112 to advance elongated member 116 distally through vasculature, and so that catheter 112 may resist kinking when traversing around a tight turn in the vasculature.

Elongated member 116 may have any suitable shape, such as a tubular body or a paddle-like shape. Elongated member 116 may be constructed using any suitable materials. In some examples, elongated member 116 may include one or more polymeric materials, for example, polyamide, polyimide, polyether block amide copolymer sold under the trademark PEBAX, polyethylene terephthalate (PET), polypropylene, aliphatic, polycarbonate-based thermoplastic polyurethane, or a polyether ether ketone (PEEK) polymer that provides elongated member 116 with a predetermined flexibility. The polymeric materials may be extruded as one or more solid or hollow tubes to form elongated member 116.

In some examples, at least a portion of an outer surface of elongated member 116 may include one or more coatings, such as, but not limited to, an anti-thrombogenic coating, which may help reduce the formation of thrombi in vitro, an anti-microbial coating, and/or a lubricating coating. In some examples, the entire working length of elongated member 116 may be coated with the hydrophilic coating. In other examples, only a portion of the working length of elongated member 116 coated with the hydrophilic coating. This may provide a length of elongated member 116 distal to hub portion 118 with which the clinician may grip elongated member 116, e.g., to rotate elongated member 116 or push elongated member 116 through vasculature. In some examples, the entire working length of elongated member 116 or portions thereof may include a lubricious outer surface, e.g., a lubricious coating. The lubricating coating may be configured to reduce static friction and/or kinetic friction at a surface of elongated member 116 as elongated member 116 is advanced through the vasculature.

The proximal portion of elongated member 116 may be received within hub portion 118 and can be mechanically connected to hub portion 18 via an adhesive, welding, or another suitable technique or combination of techniques. Hub portion 118 may serve as a handle for catheter 112 allowing the clinician to grasp catheter 112 at hub portion 118 and advance elongated member 116 through vasculature of a patient. In some examples, catheter 112 can include another structure in addition or instead of hub portion 118. For example, catheter 112 or hub portion 118 may include one or more luers or other mechanisms (e.g., access ports) for establishing connections between catheter 112 and other devices. Additionally, or alternatively, catheter 112 may include a strain relief body (not shown), which may be a part of hub portion 118 or may be separate from hub portion 118 to alleviate potential strain of kinking of elongated member 116 near its proximal end.

In some examples, system 100 includes an electrode 132 (for example, a dispersive electrode or a patient return electrode) electrically connected to generator and sensing device 150 and attached to the exterior of the patient. In some examples, generator and sensing device 150 may be configured to deliver therapy via electrodes 122 and electrode 132 in a monopolar configuration, or by a bipolar or a multipolar configuration of multiple electrodes, or a combination of monopolar and bipolar stimulation.

A secondary input 136, for example, a foot pedal, may be connected (e.g., pneumatically connected or electrically connected) to generator and sensing device 150 to allow the clinician to initiate, terminate and, optionally, adjust various operational characteristics of generator and sensing device 150, including, but not limited to, power delivery. System 100 may also include a remote control device (not shown) that can be positioned in a sterile field and operably coupled to one or both of catheter 112 or generator and sensing device 150. The remote control device may be configured to allow for selectively turning on/off electrodes of electrodes 122. In other examples, the remote control device may be built into hub portion 118.

In some examples, system 100 includes programmer 140, and generator and sensing device 150 may be connected to programmer 140. Generator and sensing device 150 can be configured to receive one or more therapy parameter values with which generator generates and delivers an electrical signal to electrodes 122 via programmer 140. In some examples, the electrical signal comprises a radiofrequency (RF) stimulus configured to ablate tissue at a target treatment site. In some examples, generator and sensing device 150 or programmer 140 may include one or more evaluation or feedback modules to provide feedback to the clinician before, during, and/or after therapy.

Programmer 140 and generator and sensing device 150 may form computing system 120. Programmer 140 may include user interface 148, processing circuitry 142, user interface 148, and memory 144. In some examples, processing circuitry 142 and memory 144 may perform functions described with respect to processing circuitry 152 and memory 154 of generator and sensing device 150.

A user, either a clinician or patient, may interact with processing circuitry 142 through user interface 148. User interface 148 may include a display, such as a liquid crystal display (LCD), light-emitting diode (LED) display, or other screen, to present information related to stimulation therapy, and buttons or a pad to provide input to programmer 140. In examples in which user interface 148 requires a 3D environment, the user interface may support 3D environments such as a holographic display, a stereoscopic display, an autostereoscopic display, a head-mounted 3D display, or any other display that is capable of presenting a 3D image to the user. Buttons of user interface 148 may include an on/off switch, plus and minus buttons to zoom in or out or navigate through options, a select button to pick or store an input, and pointing device, e.g. a mouse, trackball, or stylus. Other input devices may be a wheel to scroll through options or a touch pad to move a pointing device on the display. In some examples, the display may be a touch screen that enables the user to select options directly from the display screen.

Memory 144 includes computer-readable instructions that, when executed by processing circuitry 142, causes programmer 140 to perform various functions. Processing circuitry 142 may include any one or more microprocessors, controllers, digital signal processors (DSPs), application specific integrated circuits (ASICs), field-programmable gate arrays (FPGAs), or equivalent discrete or integrated digital or analog logic circuitry, and the functions attributed to processing circuitry 142 herein may be embodied as software, firmware, hardware or any combination thereof.

Memory 144 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media. Memory 144 may store any suitable information for determining apposition information associated with catheter 112. Memory 144 may also store operating instructions with which processing circuitry 142 controls the operation of programmer 140.

In accordance with aspects of this disclosure, generator and sensing device 150 may use electrodes 122 to determine the apposition of catheter 112 by performing modulation (e.g., amplitude modulation but other modulation types are possible) of signals transmitted by one or more of electrodes 122. Processing circuitry 152 of generator and sensing device 150 and/or processing circuitry 142 of programmer 140 may be configured to modulate a carrier signal to generate a modulated signal that carries a message signal for transmission by a first electrode of electrodes 122 of catheter 112. Oscillator 156 of generator and sensing device 150 and/or oscillator 146 of programmer 140 may be configured to generate a carrier signal, such as in the form of a sinusoidal signal, and processing circuitry 152 and/or processing circuitry 142 may be configured to modulate the carrier signal to carry the message signal, such as by performing amplitude modulation of the carrier signal to generate a modulated signal that carries the message signal.

For example, processing circuitry 142 and/or processing circuitry 152 modulates the amplitude of the carrier signal to generate a modulated signal where the envelope of the modulated signal carries the message signal. The carrier signal may have a carrier frequency in the radio band, which may be between about 420 kilohertz (kHz) to about 480 kHz, or may have a carrier frequency up to 1 Megahertz (MHz). The message signal may be a 1 Volt peak-to-peak sinusoidal signal. The modulated signal may have a modulation frequency that may be between about 15 kHz to about 50 kHz.

Processing circuitry 152 may be configured to communicate with drive circuitry 158 to electrically drive an electrode of electrodes 122 to transmit the modulated signal. That is, drive circuitry 158 may be configured to drive an electrode of electrodes 122 to output the modulated signal in the form of electrical energy, RF energy, and the like. For example, processing circuitry 152 may be configured to cause drive circuitry 158 to drive electrode 122A to output the modulated signal. An electrode, such as electrode 122A, of catheter 112 may output the modulated signal by outputting electrical energy, RF energy, and the like having characteristics of the modulated signal. That is, an electrode may output electrical energy, RF energy, and the like having the same amplitude variation, carrier frequency, modulation index, and the like of the modulated signal.

As the first electrode of catheter 112 transmits the modulated signal, other electrodes of electrodes 122 of catheter 112 may receive the modulated signal being transmitted by the first electrode. Because the modulated signal transmitted by the first electrode may suffer from several types of loss, such as attenuation, dispersion, distortion, reflection, scattering, absorption, and the like, the modulated signal that is received by each of the other electrodes may suffer from degradation of amplitude, power, and/or signal quality due to traveling through a medium compared to the modulated signal being transmitted by the first electrode.

Each of the other electrodes of electrodes 122 of catheter 112 may, in response to receiving the modulated signal, send an indication of the received modulated signal to generator and sensing device 150. In the example where electrode 122A transmits the modulated signal, electrodes 122B-122E may each receive the modulated signal, and each of electrodes 122B-122E may send an indication of the modulated signal received by the corresponding electrode to generator and sensing device 150. The indication of the modulated signal sent by each of electrodes 122B-122E may be in analog or in digital form and may indicate the characteristics of the modulated signal received by the corresponding electrode, such as the amplitude variation, carrier frequency, modulation index, and the like of the received modulated signal.

Processing circuitry 152 of generator and sensing device 150 and/or processing circuitry 142 of programmer 140 may be configured to, in response to modulating a carrier signal to generate a modulated signal that carries a message for transmission by a first electrode of electrodes 122 of catheter 112, receive an indication of the modulated signal received by a second electrode of electrodes 122 of catheter 112. As described above, when an electrode of catheter 112 transmits the modulated signal, other electrodes of electrodes 122 of catheter 112 may receive the modulated signal being transmitted by the electrode, where the modulated signal received by each of the other electrodes may have suffered a loss compared with the modulated signal transmitted by the electrode. Such loss may be due to the distance between the electrode transmitting the modulated signal and each of the other electrodes of electrodes 122 of catheter 112.

An electrode that is closest in distance to the electrode transmitting the modulated signal may receive the modulated signal in a form that suffers the smallest amount of loss compared to the modulated signal that is received by other electrodes of electrodes 122 of catheter 112. For example, if electrode 122A is transmitting the modulated signal, electrode 122B may receive the modulated signal that suffers the smallest amount of loss compared to the modulated signal received by electrodes 122C-122E.

Processing circuitry 152 of generator and sensing device 150 and/or processing circuitry 142 of programmer 140 may therefore be configured to receive an indication of the modulated signal received by electrode 122B, and processing circuitry 152 and/or processing circuitry 142 may be configured to demodulate the modulated signal received by electrode 122B to extract a demodulated message carried by the modulated signal. For example, catheter 112 may be configured to communicate with generator and sensing device 150 to send an indication of the modulated signal received by electrode 122B, such as in digital or analog form. The indication of the modulated signal received by electrode 122B may indicate the amplitude variation, carrier frequency, modulation index, and the like of the received modulated signal. In some examples, generator and sensing device 150 may be configured to send the indication of the modulated signal received by electrode 122B to programmer 140.

Processing circuitry 152 and/or processing circuitry 142 may be configured to demodulate the modulated signal received by electrode 122B at the known frequency of the modulated signal transmitted by electrode 122A. For example, if the modulated signal is driven by drive circuitry 158 to transmit the modulated signal at 18 kHz, processing circuitry 152 and/or processing circuitry 142 may demodulate the signal received by electrode 122B at 18 kHz to extract the demodulated message.

Processing circuitry 152 of generator and sensing device 150 and/or processing circuitry 142 of programmer 140 may be configured to determine apposition information associated with catheter 112 based at least in part on the demodulated message extracted from the signal. Processing circuitry 152 and/or processing circuitry 142 may be configured to determine, based at least in part on the demodulated message, whether catheter 112 makes contact against a target tissue site, such as an organ tissue, arterial walls, venous walls, or other structures of the patient, or whether catheter 112 is disposed in the blood stream (i.e., catheter 112 is not making contact against the target tissue site) based at least in part on the demodulated message.

Processing circuitry 152 and/or processing circuitry 142 may be configured to determine apposition information associated with catheter 112 by comparing the demodulated message recovered from the modulated signal received by the second electrode (e.g., electrode 122B) with the message carried by the modulated signal that is transmitted by the first electrode (e.g., electrode 122A). When the demodulated signal travels through different mediums, the amount of loss suffered by the demodulated signal may depend upon the medium through which the demodulated signal travels. For example, a demodulated signal may suffer a greater loss when traveling through a wall of an organ or a vessel, such as when catheter 112 is in contact with a wall of an organ or a vessel, compared with the demodulated signal traveling through fat or blood, such as when catheter is not in contact with the wall of an organ or a vessel. As such, processing circuitry 152 and/or processing circuitry 142 may be configured to measure the amount of loss suffered by the modulated signal as the modulated signal travels from a first electrode (e.g., electrode 122A) to a second electrode (e.g., electrode 122B) and may use the measured amount of loss to determine whether catheter 112 is in contact with a wall of an organ or a vessel of a patient.

In some examples, processing circuitry 152 and/or processing circuitry 142 may be configured to compare the demodulated message recovered from the modulated signal received by the second electrode (e.g., electrode 122B) with the message carried by the modulated signal as transmitted by the first electrode (e.g., electrode 122A) to determine the signal loss of the demodulated signal due to traveling through a medium from the first electrode to the second electrode. For example, processing circuitry 152 and/or processing circuitry 142 may be configured to compare the demodulated message with the message to determine the recovery rate of the message in the demodulated message. Processing circuitry 152 and/or processing circuitry 142 may therefore be configured to determine apposition information associated with catheter 112 based on the recovery rate of the message in the demodulated message.

The recovery rate of a signal may refer to the ability to accurately reconstruct the signal from a distorted or degraded version of the signal and may be expressed as a percentage or a ratio of the quality of the recovered signal to the quality of the original signal. For example, if the recovered signal is 90% as good as the original signal, the recovery rate of the signal may be expressed as 0.9 or 90%.

Processing circuitry 152 and/or processing circuitry 142 may therefore be configured to determine the recovery rate of the message carried by the modulated signal as transmitted by the first electrode from the demodulated message recovered from the modulated signal received by the second electrode. For example, processing circuitry 152 and/or processing circuitry 142 may be configured to determine that catheter 112 is in contact with a target tissue site, such as an organ tissue, arterial walls, venous walls, or other structures of the patient if the recovery rate of the message from the demodulated message is about 50%. In another example, processing circuitry 152 and/or processing circuitry 142 may be configured to determine that catheter 112 is not in contact with the target tissue site if the recovery rate of the message from the demodulated message is about 50%.

Processing circuitry 152 and/or processing circuitry 142 may be configured to, in response to determining appositional information associated with catheter 112 output an indication of the appositional information associated with catheter 112. For example, processing circuitry 142 may be configured to output, via user interface 148, an indication of whether catheter 112 is in contact with the target tissue site. For example, processing circuitry 142 may output, for display at a display device of user interface 148, a visual indication of whether catheter 112 is in contact with the target tissue site. In another example, processing circuitry 142 may output, at an audio output device (e.g., a speaker) of user interface 148, an audible indication of whether catheter 112 is in contact with the target tissue site.

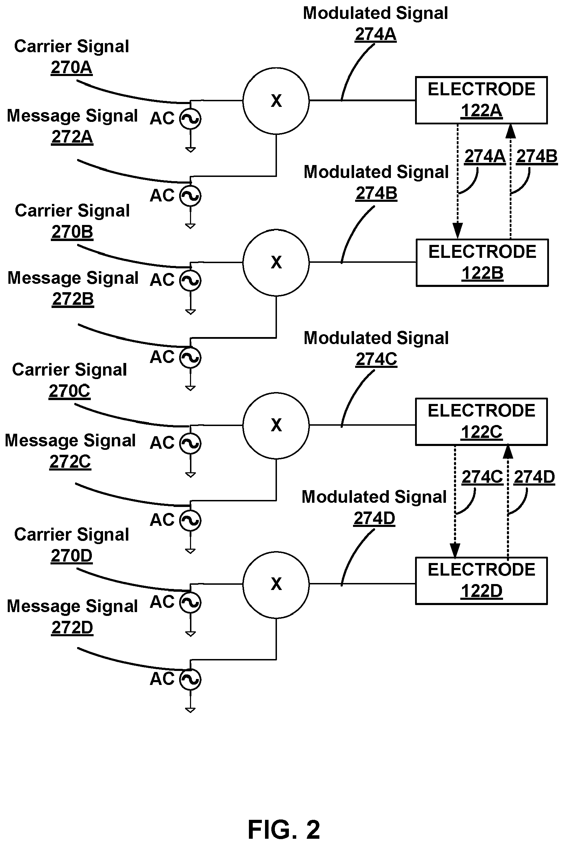

FIG. 2 illustrates using pairs of electrodes 122 of catheter 112 to determine whether catheter 112 is in contact with a target tissue site. While FIG. 1 illustrates using a single pair of electrodes (e.g., electrodes 122A and 122B of catheter 112) to determine whether catheter 112 is in contact with a target tissue site, computing system 120 (i.e., programmer 140 and/or generator and sensing device 150) may, in some examples, use a plurality of electrode pairs of electrodes 122 of catheter 112 to determine whether catheter 112 is in contact with a target tissue site. A pair of electrodes that are closest to each other may be paired up so that a first electrode of the pair of electrodes may transmit a modulated signal and a second electrode of the pair of electrodes may receive the modulated signal transmitted by the first electrode.

As shown in FIG. 2, electrode 122A may be paired with electrode 122B and electrode 122C may be paired with electrode 122D. Processing circuitry 142 and/or processing circuitry 152 of computing system 120 may be configured to perform amplitude modulation of carrier signal 270A to generate modulated signal 274A that carries message signal 272A. Electrode 122A of catheter 112 may transmit modulated signal 274A, and electrode 122B paired up with electrode 122A may receive modulated signal 274A. Processing circuitry 142 and/or processing circuitry 152 may be configured to demodulate modulated signal 274A received by electrode 122B to extract a demodulated message and may compare the demodulated message with message signal 272A to determine whether electrode 122A is in contact against a target tissue site, such as an organ tissue, arterial walls, venous walls, or other structures of the patient.

Processing circuitry 142 and/or processing circuitry 152 may be configured to perform amplitude modulation of carrier signal 270B to generate modulated signal 274B that carries message signal 272B. Electrode 122B of catheter 112 may transmit modulated signal 274B, and electrode 122A paired up with electrode 122B may receive modulated signal 274B. Processing circuitry 142 and/or processing circuitry 152 may be configured to demodulate modulated signal 274B received by electrode 122A to extract a demodulated message and may compare the demodulated message with message signal 272B to determine whether electrode 122B is in contact against a target tissue site, such as an organ tissue, arterial walls, venous walls, or other structures of the patient.

Processing circuitry 142 and/or processing circuitry 152 may be configured to perform amplitude modulation of carrier signal 270C to generate modulated signal 274C that carries message signal 272C. Electrode 122C of catheter 112 may transmit modulated signal 274C, and electrode 122D paired up with electrode 122C may receive modulated signal 274C. Processing circuitry 142 and/or processing circuitry 152 may be configured to demodulate modulated signal 274C received by electrode 122D to extract a demodulated message and may compare the demodulated message with message signal 272C to determine whether electrode 122C is in contact against a target tissue site, such as an organ tissue, arterial walls, venous walls, or other structures of the patient.

Processing circuitry 142 and/or processing circuitry 152 may be configured to perform amplitude modulation of carrier signal 270D to generate modulated signal 274D that carries message signal 272D. Electrode 122D of catheter 112 may transmit modulated signal 274D, and electrode 122C paired up with electrode 122D may receive modulated signal 274D. Processing circuitry 142 and/or processing circuitry 152 may be configured to demodulate modulated signal 274D received by electrode 122C to extract a demodulated message and may compare the demodulated message with message signal 272D to determine whether electrode 122D is in contact against a target tissue site, such as an organ tissue, arterial walls, venous walls, or other structures of the patient. In this way, processing circuitry 142 and/or processing circuitry 152 may be able to determine whether each of a plurality of electrodes 122A-D of catheter 112 contacts the target tissue site of a patient.

In some examples, each of carrier signals 270A-270D may have the same frequency, which may be within a frequency range of 420 kHz to 480 kHz, or may be within another frequency range. In some examples, two or more of carrier signals 270A-270D may have different frequencies. For example, carrier signal 270A may have a frequency of 460 kHz while carrier signal 270B may have a frequency of 470 kHz.

Message signals 272A-272D may be separated by a discernible frequency per the capabilities of processing circuitry 142 and/or processing circuitry 152 to demodulate such message signals 272A-272D. That is, message signals 272A-272D may each have a different frequency to ensure that an electrode is not obtaining a message from a signal driven by the electrode itself. In some examples, message signals 272A-272D may be separated by 25 hertz, 50 hertz, 100 hertz, 1 kHz, and the like. For example, message signal 272A may have a frequency of 18 kHz, message signal 272B may have a frequency of 19 kHz, message signal 272C may have a frequency of 20 kHz, and message signal 272D may have a frequency of 21 kHz. In these examples, the frequencies of message signals 272A-272D may each be separated by 1 kHz.

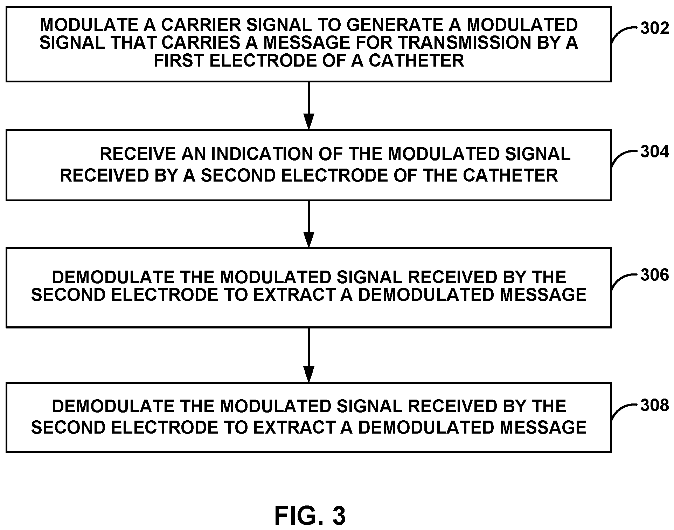

FIG. 3 is a flow diagram illustrating an example technique for determining appositional information associated with a catheter 112. FIG. 3 is described with respect to FIGS. 1 and 2.

As shown in FIG. 3, processing circuitry 142 of programmer 140 and/or processing circuitry 152 of generator and sensing device 150 may modulate a carrier signal to generate a modulated signal that carries a message for transmission by a first electrode 122A of a catheter 112 (302). Processing circuitry 142 and/or processing circuitry 152 may receive an indication of the modulated signal received by a second electrode 122B of the catheter 112 (304). Processing circuitry 142 and/or processing circuitry 152 may demodulate the modulated signal received by the second electrode 122B to extract a demodulated message (306). Processing circuitry 142 and/or processing circuitry 152 may determine whether the first electrode 122A contacts a target tissue site of a patient based at least in part on the demodulated message (308).

The techniques described in this disclosure may be implemented, at least in part, in hardware, software, firmware, or any combination thereof. For example, various aspects of the described techniques may be implemented within processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also perform one or more of the techniques of this disclosure.

Such hardware, software, and firmware may be implemented within the same device or within separate devices to support the various techniques described in this disclosure. In addition, any of the described instructions, units, modules or components may be implemented together or separately as discrete but interoperable logic devices. Depiction of different features as modules or units is intended to highlight different functional aspects and does not necessarily imply that such modules or units must be realized by separate hardware, firmware, or software components. Rather, functionality associated with one or more modules or units may be performed by separate hardware, firmware, or software components, or integrated within common or separate hardware, firmware, or software components.

The techniques described in this disclosure may also be embodied or encoded in a computer system-readable medium, such as a computer system-readable storage medium, containing instructions. Instructions embedded or encoded in a computer system-readable medium, including a computer system-readable storage medium, may cause one or more programmable processors, or other processors, to implement one or more of the techniques described herein, such as when instructions included or encoded in the computer system-readable medium are executed by the processing circuitry. Computer system readable storage media may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or other computer system readable media. In some examples, an article of manufacture may comprise one or more computer system-readable storage media, for example, non-transitory computer system-readable storage media.

Various examples have been described. These and other examples are within the scope of the following claims.

Example 1. A method comprising: modulating, by processing circuitry, a carrier signal to generate a modulated signal that carries a message for transmission by a first electrode of a catheter; receiving, by the processing circuitry, an indication of the modulated signal received by a second electrode of the catheter; demodulating, by the processing circuitry, the modulated signal received by the second electrode to extract a demodulated message; and determining, by the processing circuitry, whether the first electrode contacts a target tissue site of a patient based at least in part on the demodulated message.

Example 2. The method of Example 1, wherein the modulated signal comprises an ablation signal.

Example 3. The method of any of Examples 1 and 2, further comprising: outputting, by the processing circuitry, an indication of whether the first electrode contacts the target tissue site of the patient.

Example 4. The method of any of Examples 1-3, wherein the target tissue site of the patient is on at least one of: an organ tissue, an arterial wall, or a venous wall of the patient.

Example 5. The method of any of Examples 1-4, wherein determining whether the first electrode contacts the target tissue site of the patient further comprises: comparing, by the processing circuitry, the demodulated message with the message carried by the modulated signal to determine whether the first electrode contacts the target tissue site of the patient.

Example 6. The method of Example 5 wherein comparing the demodulated message with the message carried by the modulated signal further comprises: comparing, by the processing circuitry, the demodulated message with the message carried by the modulated signal to determine a recovery rate of the message; and determining, by the processing circuitry, whether the first electrode contacts the target tissue site of the patient based at least in part on the recovery rate of the message.

Example 7. The method of any of Examples 1-6, further comprising: modulating, by the processing circuitry, a second carrier signal to generate a second modulated signal that carries a second message for transmission by the second electrode; receiving, by the processing circuitry, an indication of the second modulated signal received by the first electrode of the catheter; demodulating, by the processing circuitry, the second modulated signal received by the first electrode to extract a second demodulated message; and determining, by the processing circuitry, whether the second electrode contacts the target tissue site of the patient based at least in part on the second demodulated message.

Example 8. The method of Example 7, wherein the carrier signal has a first carrier frequency and the second carrier signal has a second carrier frequency that is the same as the first carrier frequency.

Example 9. The method of Example 8, wherein the first carrier frequency and the second carrier frequency are in a radio band.

Example 10. The method of any of Examples 7-9, wherein the message has a first frequency and the second message has a second frequency different from the first frequency.

Example 11. The method of Example 10, wherein the first frequency and the second frequency are separated by a discernable frequency.

Example 12. The method of any of Examples 1-11, wherein modulating the carrier signal further comprises: modulating, by the processing circuitry, an amplitude of the carrier signal to generate the modulated signal that carries the message.

Example 13. A system comprising: memory; processing circuitry configured to: modulate a carrier signal to generate a modulated signal that carries a message for transmission by a first electrode of a catheter; receive an indication of the modulated signal received by a second electrode of the catheter; demodulate the modulated signal received by the second electrode to extract a demodulated message; and determine whether the first electrode contacts a target tissue site of a patient based at least in part on the demodulated message.

Example 14. The system of Example 13, wherein the modulated signal comprises an ablation signal.

Example 15. The system of any of Examples 13 and 14, wherein the processing circuitry is further configured to: output an indication of whether the first electrode contacts the target tissue site of the patient.

Example 16. The system of any of Examples 13-15, wherein the target tissue site of the patient is on at least one of an organ tissue, an arterial wall, or a venous wall of the patient.

Example 17. The system of any of Examples 13-16, wherein to determine whether the first electrode contacts the target tissue site of the patient, the processing circuitry is further configured to: compare the demodulated message with the message carried by the modulated signal to determine whether the first electrode contacts the target tissue site of the patient.

Example 18. The system of Example 17 wherein to compare the demodulated message with the message carried by the modulated signal, the processing circuitry is further configured to: compare the demodulated message with the message carried by the modulated signal to determine a recovery rate of the message; and determine whether the first electrode contacts the target tissue site of the patient based at least in part on the recovery rate of the message.

Example 19. The system of any of Examples 13-18, wherein the processing circuitry is further configured to: modulate a second carrier signal to generate a second modulated signal that carries a second message for transmission by the second electrode;
receive an indication of the second modulated signal received by the first electrode of the catheter; demodulate the second modulated signal received by the first electrode to extract a second demodulated message; and demodulate whether the second electrode contacts the target tissue site of the patient based at least in part on the second demodulated message.

Example 20. The system of Example 19, wherein the carrier signal has a first carrier frequency and the second carrier signal has a second carrier frequency that is the same as the first carrier frequency.

Example 21. The system of Example 20, wherein the first carrier frequency and the second carrier frequency are in a radio band.

Example 22. The system of any of Examples 19-21, wherein the message has a first frequency and the second message has a second frequency different from the first frequency.

Example 23. The system of Example 22, wherein the first frequency and the second frequency are separated by a a discernable frequency.

Example 24. The system of any of Examples 13-23, wherein to modulate the carrier signal, the processing circuitry is further configured to: modulate an amplitude of the carrier signal to generate the modulated signal that carries the message.

Example 25. An apparatus comprising means for performing the method of any of Examples 1-12.

Example 26. A non-transitory computer readable storage medium comprising instructions that, when executed by processing circuitry, cause the processing circuitry to perform the method of any of Examples 1-12.

Further disclosed herein is the subject-matter of the following clauses:
1. A system comprising:
   memory;
   processing circuitry configured to:
      modulate a carrier signal to generate a modulated signal that carries a message for transmission by a first electrode of a catheter;
      receive an indication of the modulated signal received by a second electrode of the catheter;
      demodulate the modulated signal received by the second electrode to extract a demodulated message; and
      determine whether the first electrode contacts a target tissue site of a patient based at least in part on the demodulated message.
2. The system of clause 1, wherein the modulated signal comprises an ablation signal.
3. The system of any of clauses 1 and 2, wherein the processing circuitry is further configured to:
   output an indication of whether the first electrode contacts the target tissue site of the patient.
4. The system of any of clauses 1-3, wherein the target tissue site of the patient is on at least one of: an organ tissue, an arterial wall, or a venous wall of the patient.
5. The system of any of clauses 1-4, wherein to determine whether the first electrode contacts the target tissue site of the patient, the processing circuitry is further configured to:
   compare the demodulated message with the message carried by the modulated signal to determine whether the first electrode contacts the target tissue site of the patient.
6. The system of clause 5 wherein to compare the demodulated message with the message carried by the modulated signal, the processing circuitry is further configured to:
   compare the demodulated message with the message carried by the modulated signal to determine a recovery rate of the message; and
   determine whether the first electrode contacts the target tissue site of the patient based at least in part on the recovery rate of the message.
7. The system of any of clauses 1-6, wherein the processing circuitry is further configured to:
   modulate a second carrier signal to generate a second modulated signal that carries a second message for transmission by the second electrode;
   receive an indication of the second modulated signal received by the first electrode of the catheter;
   demodulate the second modulated signal received by the first electrode to extract a second demodulated message; and
   demodulate whether the second electrode contacts the target tissue site of the patient based at least in part on the second demodulated message.
8. The system of clause 7, wherein the carrier signal has a first carrier frequency and the second carrier signal has a second carrier frequency that is the same as the first carrier frequency.
9. The system of clause 8, wherein the first carrier frequency and the second carrier frequency are in a radio band.
10. The system of any of clauses 7-9, wherein the message has a first frequency and the second message has a second frequency different from the first frequency.
11. The system of clause 10, wherein the first frequency and the second frequency are separated by a a discernable frequency.
12. The system of any of clauses 1-11, wherein to modulate the carrier signal, the processing circuitry is further configured to:
   modulate an amplitude of the carrier signal to generate the modulated signal that carries the message.
13. A method comprising:
   modulating, by processing circuitry, a carrier signal to generate a modulated signal that carries a message for transmission by a first electrode of a catheter;
   receiving, by the processing circuitry, an indication of the modulated signal received by a second electrode of the catheter;
   demodulating, by the processing circuitry, the modulated signal received by the second electrode to extract a demodulated message; and
   determining, by the processing circuitry, whether the first electrode contacts a target tissue site of a patient based at least in part on the demodulated message.
14. A non-transitory computer readable storage medium comprising instructions that, when executed by processing circuitry, cause the processing circuitry to:
   modulate a carrier signal to generate a modulated signal that carries a message for transmission by a first electrode of a catheter;
   receive an indication of the modulated signal received by a second electrode of the catheter;
   demodulate the modulated signal received by the second electrode to extract a demodulated message; and
   determine whether the first electrode contacts a target tissue site of a patient based at least in part on the demodulated message.

## Claims

1. A system comprising:
memory;
processing circuitry configured to:
modulate a carrier signal to generate a modulated signal that carries a message for transmission by a first electrode of a catheter;
receive an indication of the modulated signal received by a second electrode of the catheter;
demodulate the modulated signal received by the second electrode to extract a demodulated message; and
determine whether the first electrode contacts a target tissue site of a patient based at least in part on the demodulated message.

2. The system of claim 1, wherein the modulated signal comprises an ablation signal.

3. The system of any of claims 1 and 2, wherein the processing circuitry is further configured to:
output an indication of whether the first electrode contacts the target tissue site of the patient.

4. The system of any of claims 1-3, wherein the target tissue site of the patient is on at least one of: an organ tissue, an arterial wall, or a venous wall of the patient.

5. The system of any of claims 1-4, wherein to determine whether the first electrode contacts the target tissue site of the patient, the processing circuitry is further configured to:
compare the demodulated message with the message carried by the modulated signal to determine whether the first electrode contacts the target tissue site of the patient.

6. The system of claim 5 wherein to compare the demodulated message with the message carried by the modulated signal, the processing circuitry is further configured to:
compare the demodulated message with the message carried by the modulated signal to determine a recovery rate of the message; and
determine whether the first electrode contacts the target tissue site of the patient based at least in part on the recovery rate of the message.

7. The system of any of claims 1-6, wherein the processing circuitry is further configured to:
modulate a second carrier signal to generate a second modulated signal that carries a second message for transmission by the second electrode;
receive an indication of the second modulated signal received by the first electrode of the catheter;
demodulate the second modulated signal received by the first electrode to extract a second demodulated message; and
demodulate whether the second electrode contacts the target tissue site of the patient based at least in part on the second demodulated message.

8. The system of claim 7, wherein the carrier signal has a first carrier frequency and the second carrier signal has a second carrier frequency that is the same as the first carrier frequency.

9. The system of claim 8, wherein the first carrier frequency and the second carrier frequency are in a radio band.

10. The system of any of claims 7-9, wherein the message has a first frequency and the second message has a second frequency different from the first frequency.

11. The system of claim 10, wherein the first frequency and the second frequency are separated by a a discernable frequency.

12. The system of any of claims 1-11, wherein to modulate the carrier signal, the processing circuitry is further configured to:
modulate an amplitude of the carrier signal to generate the modulated signal that carries the message.

13. A method comprising:
modulating, by processing circuitry, a carrier signal to generate a modulated signal that carries a message for transmission by a first electrode of a catheter;
receiving, by the processing circuitry, an indication of the modulated signal received by a second electrode of the catheter;
demodulating, by the processing circuitry, the modulated signal received by the second electrode to extract a demodulated message; and
determining, by the processing circuitry, whether the first electrode contacts a target tissue site of a patient based at least in part on the demodulated message.

14. A non-transitory computer readable storage medium comprising instructions that, when executed by processing circuitry, cause the processing circuitry to:
modulate a carrier signal to generate a modulated signal that carries a message for transmission by a first electrode of a catheter;
receive an indication of the modulated signal received by a second electrode of the catheter;
demodulate the modulated signal received by the second electrode to extract a demodulated message; and
determine whether the first electrode contacts a target tissue site of a patient based at least in part on the demodulated message.
